(19) Europäisches Patentamt
European Patent Office
Office européen des brevets

(11) **EP 4 198 455 A1**

(12) **EUROPEAN PATENT APPLICATION**

(43) Date of publication:
**21.06.2023 Bulletin 2023/25**

(21) Application number: **22210398.8**

(22) Date of filing: **29.11.2022**

(51) International Patent Classification (IPC):
**G01C 21/34** (2006.01) **B60W 40/09** (2012.01)
**B60W 50/00** (2006.01) **G01C 21/36** (2006.01)

(52) Cooperative Patent Classification (CPC):
**G01C 21/3484; B60W 40/09; B60W 50/0098;
G01C 21/3641**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(30) Priority: **16.12.2021 US 202117553348**

(71) Applicant: **VOLKSWAGEN AG
38440 Wolfsburg (DE)**

(72) Inventors:
• **Winther, Morten Troestrup
Sunnyvale, 94087 (US)**
• **Tulit, Vince
San Mateo, 94403 (US)**
• **Jaffe, Hannah
Los Angeles, 90004 (US)**
• **Olcay, Enes
Redwood City, 94063 (US)**
• **Dhar, Sagnik
San Francisco, 94131 (US)**

(54) **DYNAMIC MODALITY-BASED VEHICLE NAVIGATION**

(57) Technologies and techniques for controlling modalities for vehicle navigation. A navigation system may be configured to transmit navigation data and communicate the navigation data in a vehicle using a plurality of modalities. One or more sensors provide (604) data associated with at least one of (i) sensor data associated with a vehicle interior environment, (ii) sensor data associated with a physiological state of a driver, and/or (iii) driving condition data. A processor, may be configured to process (606) at least one of the sensor data, driving condition data and/or navigation data to determine one or more modality values, wherein the processor is configured to activate and/or deactivate (608) at least one of the plurality of modalities based on the one or more modality values.

FIG. 6

**Description**

The present disclosure relates to vehicle navigation. More specifically, the present disclosure is directed to vehicle navigation using dynamically adjustable modalities to communicate navigation information.

An automotive navigation system is part of vehicle controls (or a third-party add-on) used to find direction in an automobile. A navigation system typically uses a satellite navigation device to get position data, which is then correlated to a position on a road. When directions are needed, routing can be calculated. On-the-fly traffic information can also be used to adjust the route. Dead reckoning, which uses distance data from sensors attached to the drivetrain, a gyroscope, and an accelerometer, can also be used for greater reliability, as GPS signal loss and/or multipath can occur due to urban canyons or tunnels. Mathematically, automotive navigation may be considered a system based on the "shortest path problem" within graph theory, which examines how to identify a path that best meets some criteria (shortest, cheapest, fastest, etc.) between two points in a large network.

During operation, current navigation systems typically are unable or limited in their ability to communicate directions and/or instructions to a driver selectively across different modalities (e.g., graphic user interface (GUI), heads-up display, voice commands, etc.). Moreover, current navigation systems do not have sufficient abilities to take into account contextual data regarding a driver's driving environment and/or familiarity with a given navigational path. As a result, conventional navigation systems may be overly-intrusive to a driver when providing turn-by-turn instructions, resulting in potential distractions, and, as a result, hazards, to the driver.

The invention is as set out in the appended independent claims. Advantageous refinements are defined in the dependent claims.

Various apparatus, systems, and methods are disclosed herein relating to controlling operation of a vehicle. In some examples, a system for controlling modalities for vehicle navigation is disclosed, comprising: a navigation system configured to transmit navigation data and communicate the navigation data in a vehicle using a plurality of modalities; one or more sensors configured to provide data associated with at least one of (i) sensor data associated with a vehicle interior environment, (ii) sensor data associated with a physiological state of a driver, and/or (iii) driving condition data; and a processor, operatively coupled to the one or more sensors and the navigation system, wherein the processor is configured to process at least one of the sensor data, driving condition data, and/or navigation data to determine one or more modality values, wherein the processor is configured to activate and/or deactivate at least one of the plurality of modalities based on the one or more modality values.

In some examples, a method is disclosed for controlling modalities for vehicle navigation, comprising: receiving navigation data in a navigation system configured to communicate the navigation data in a vehicle using a plurality of modalities; receiving data associated with at least one of (i) sensor data associated with a vehicle interior environment, (ii) sensor data associated with a physiological state of a driver, and/or (iii) driving condition data; and processing, via a processor, at least one of the sensor data, driving condition data, and/or navigation data to determine one or more modality values; activating and/or deactivating at least one of the plurality of modalities based on the one or more modality values.

In some examples, a method is disclosed for controlling modalities for vehicle navigation, comprising: receiving navigation data in a navigation system configured to communicate the navigation data in a vehicle using a plurality of modalities; receiving context familiarity data comprising data representing a driver's familiarity with one or more navigational route segments of the navigation data; processing, via a processor, the context familiarity data to determine a route familiarity value; and activating and/or deactivating at least one of the plurality of modalities based on the route familiarity value.

These and other aspects of the invention will become more fully understood upon a review of the detailed description, which follows. Other aspects, features, and embodiments of the present invention will become apparent to those of ordinary skill in the art, upon reviewing the following description of specific, exemplary embodiments of the present invention in conjunction with the accompanying figures. While features of the present invention may be discussed relative to certain embodiments and figures below, all embodiments of the present invention can include one or more of the advantageous features discussed herein. In other words, while one or more embodiments may be discussed as having certain advantageous features, one or more of such features may also be used in accordance with the various embodiments of the invention discussed herein. In similar fashion, while exemplary embodiments may be discussed below as device, system, or method embodiments it should be understood that such exemplary embodiments can be implemented in various devices, systems, and methods.

The present invention is illustrated by way of example and not limitation in the figures of the accompanying drawings, in which like references indicate similar elements and in which:

FIG. 1    shows an exemplary vehicle system block diagram showing multiple components and modules, together with a navigation system according to some aspects of the present disclosure;

FIG. 2    shows a block diagram illustrating a navigation system including a modality selector and user interface according to some aspects of the present disclosure;

FIG. 3A    A shows a simulated navigation map including a navigational path between a start point and end point according to some aspects of the present disclosure;

FIG. 3B    shows a simulated turn-by turn instruction setting for a navigational system having a static modality according to some aspects of the present disclosure;

FIG. 3C    shows a simulated turn-by turn instruction setting under a dynamic modality configuration according to some aspects of the present disclosure;

FIG. 4    shows a simplified diagram of a vehicle interior including a navigation system and various associated components according to some aspects of the present disclosure;

FIG. 5    shows an illustration of multiple available navigational paths, along with a selected navigational path, in which a familiarity score is calculated for configuring navigation instructions according to some aspects of the present disclosure;

FIG. 6    shows an illustrative process for dynamically controlling modalities for vehicle navigation, according to some aspects of the present disclosure; and

FIG. 7    shows another illustrative process for dynamically controlling modalities for vehicle navigation using route familiarity, according to some aspects of the present disclosure.

The figures and descriptions provided herein may have been simplified to illustrate aspects that are relevant for a clear understanding of the herein described devices, structures, systems, and methods, while eliminating, for the purpose of clarity, other aspects that may be found in typical similar devices, systems, and methods. Those of ordinary skill may thus recognize that other elements and/or operations may be desirable and/or necessary to implement the devices, systems, and methods described herein. However, because such elements and operations are known in the art, and because they do not facilitate a better understanding of the present disclosure, a discussion of such elements and operations may not be provided herein. However, the present disclosure is deemed to inherently include all such elements, variations, and modifications to the described aspects that would be known to those of ordinary skill in the art.

Exemplary embodiments are provided throughout so that this disclosure is sufficiently thorough and fully conveys the scope of the disclosed embodiments to those who are skilled in the art. Numerous specific details are set forth, such as examples of specific components, devices, and methods, to provide this thorough understanding of embodiments of the present disclosure. Nevertheless, it will be apparent to those skilled in the art that specific disclosed details need not be employed, and that exemplary embodiments may be embodied in different forms. As such, the exemplary embodiments should not be construed to limit the scope of the disclosure. In some exemplary embodiments, well-known processes, well-known device structures, and well-known technologies may not be described in detail.

The terminology used herein is for the purpose of describing particular exemplary embodiments only and is not intended to be limiting. As used herein, the singular forms "a", "an" and "the" may be intended to include the plural forms as well, unless the context clearly indicates otherwise. The terms "comprises," "comprising," "including," and "having," are inclusive and therefore specify the presence of stated features, integers, steps, operations, elements, and/or components, but do not preclude the presence or addition of one or more other features, integers, steps, operations, elements, components, and/or groups thereof. The steps, processes, and operations described herein are not to be construed as necessarily requiring their respective performance in the particular order discussed or illustrated, unless specifically identified as a preferred order of performance. It is also to be understood that additional or alternative steps may be employed.

When an element or layer is referred to as being "on", "engaged to", "connected to" or "coupled to" another element or layer, it may be directly on, engaged, connected or coupled to the other element or layer, or intervening elements or layers may be present. In contrast, when an element is referred to as being "directly on," "directly engaged to", "directly connected to" or "directly coupled to" another element or layer, there may be no intervening elements or layers present. Other words used to describe the relationship between elements should be interpreted in a like fashion (e.g., "between" versus "directly between," "adjacent" versus "directly adjacent," etc.). As used herein, the term "and/or" includes any and all combinations of one or more of the associated listed items.

Although the terms first, second, third, etc. may be used herein to describe various elements, components, regions, layers and/or sections, these elements, components, regions, layers and/or sections should not be limited by these terms. These terms may be only used to distinguish one element, component, region, layer or section from another element, component, region, layer or section. Terms such as "first," "second," and other numerical terms when used herein do not imply a sequence or order unless clearly indicated by the context. Thus, a first element, component, region, layer or section discussed below could be termed a second element, component, region, layer or section without departing from the teachings of the exemplary embodiments.

The disclosed embodiments may be implemented, in some cases, in hardware, firmware, software, or any tangibly-embodied combination thereof. The disclosed embodiments may also be implemented as instructions carried by or stored on one or more non-transitory machine-readable (e.g., computer-readable) storage medium, which may be read and executed by one or more processors. A machine-readable storage medium may be embodied as any storage device, mechanism, or other physical structure for storing or transmitting information in a form readable by a machine (e.g., a volatile or non-volatile memory, a media disc, or other media device).

In the drawings, some structural or method features may be shown in specific arrangements and/or orderings. However, it should be appreciated that such specific arrangements and/or orderings may not be required. Rather, in some embodiments, such features may be arranged in a different manner and/or order than shown in the illustrative figures. Additionally, the inclusion of a structural or method feature in a particular figure is not meant to imply that such feature is required in all embodiments and, in some embodiments, may not be included or may be combined with other features.

It will be understood that the term "module" as used herein does not limit the functionality to particular physical modules, but may include any number of tangibly-embodied software and/or hardware components. In general, a computer program product in accordance with one embodiment comprises a tangible computer usable medium (e.g., standard RAM, an optical disc, a USB drive, or the like) having computer-readable program code embodied therein, wherein the computer-readable program code is adapted to be executed by a processor (working in connection with an operating system) to implement one or more functions and methods as described below. In this regard, the program code may be implemented in any desired language, and may be implemented as machine code, assembly code, byte code, interpretable source code or the like (e.g., via Scalable Language ("Scala"), C, C++, C#, Java, Actionscript, Objective-C, Javascript, CSS, XML, etc.).

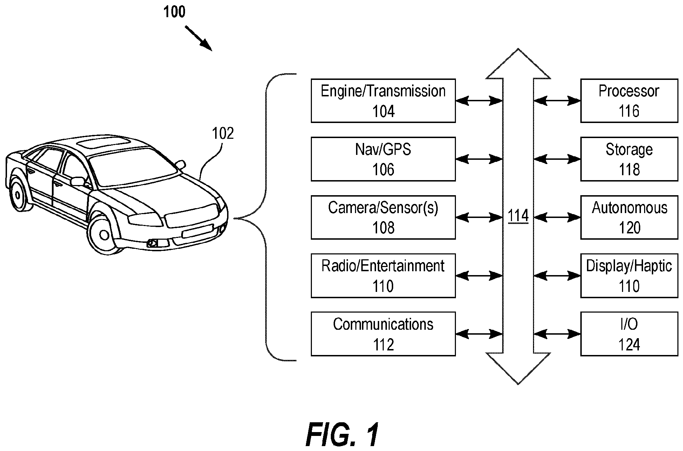

Turning to FIG. 1, the drawing illustrates an exemplary system 100 for a vehicle 102 comprising various vehicle electronics circuitries, subsystems and/or components. Engine/transmission circuitry 104 is configured to process and provide vehicle engine and transmission characteristic or parameter data, and may comprise an engine control unit (ECU) and a transmission control. For a diesel engine, circuitry 104 may provide data relating to fuel injection rate, emission control, NOx control, regeneration of oxidation catalytic converter, turbocharger control, cooling system control, and throttle control, among others. For a gasoline and/or hybrid engine, circuitry 104 may provide data relating to lambda control, on-board diagnostics, cooling system control, ignition system control, lubrication system control, fuel injection rate control, throttle control, and others. Transmission characteristic data may comprise information relating to the transmission system and the shifting of the gears, torque, and use of the clutch. Under one embodiment, an engine control unit and transmission control may exchange messages, sensor signals and control signals for any of gasoline, hybrid and/or electrical engines.

Navigation/Global positioning system (GPS) circuitry 106 provides navigation processing and location data for the vehicle 102. The camera/sensors 108 provide image or video data (with or without sound), and sensor data, which may comprise data relating to vehicle characteristic and/or parameter data, and may also provide environmental data pertaining to the vehicle, its interior and/or surroundings, such as temperature, sound, light, humidity, and the like, and may further include LiDAR, radar, image processing, and computer vision. Radio/entertainment circuitry 110 may provide data relating to audio/video media being played in vehicle 102. The radio/entertainment circuitry 110 may be integrated and/or communicatively coupled to an entertainment unit configured to play AM/FM radio, satellite radio, compact disks, DVDs, digital media, streaming media, and the like.

Communications circuitry 112 allows any of the circuitries of system 100 to communicate with each other and/or external devices via a wired connection (e.g., Controller Area Network (CAN bus), local interconnect network, etc.) or wireless protocol, such as 3G, 4G, 5G, Wi-Fi, Bluetooth, Dedicated Short Range Communications (DSRC), cellular vehicle-to-everything (C-V2X) PC5 or NR, and/or any other suitable wireless protocol. While communications circuitry 112 is shown as a single circuit, it should be understood by a person of ordinary skill in the art that communications circuitry 112 may be configured as a plurality of circuits. In some examples, communications circuitry 112 may communicate with a vehicle-to-infrastructure (V2I) system, which may be configured as a communication model that allows vehicles to share information with the components that support a country's highway system. Such components include overhead wireless (e.g., RFID) readers and cameras, traffic lights, lane markers, streetlights, signage and parking meters. V2I communication is typically wireless and bidirectional: data from infrastructure components can be delivered to the vehicle over an ad hoc network and vice versa. Similar to vehicle-to-vehicle (V2V) communication, V2I uses dedicated short-range communication (DSRC), C-V2X PC5 or C-V2X NR communication mode to transfer data. All modes may support messages in the SAE J2735 format.

Alternately or in addition, communication circuitry 112 may communicate with an intelligent transportation system (ITS), where V2I sensors can capture infrastructure data and provide vehicles with real-time advisories about such things as road conditions, traffic congestion, accidents, construction zones and parking availability. Likewise, traffic management supervision systems can use infrastructure and vehicle data to set variable speed limits and adjust traffic signal phase and timing (SPaT) to increase fuel economy and traffic flow. In some examples, circuitries 104-112 may be communicatively coupled to bus 114 for certain communication and data exchange purposes.

Vehicle 102 may further include a main processor 116 (which may be referred to as a "processing apparatus") that centrally processes and controls data communication throughout the system 100. The processor 116 may be configured as a single processor, multiple processors, or part of a processor system. In some illustrative embodiments, the processor 116 may be equipped with advanced driver assistance circuitries, navigation, sensor processing, and infotainment circuitries that allow for communication with and control of any of the circuitries in vehicle 100. Processor 116 may also be configured with a digital signal processor (DSP) for processing signals received from sensors 108 or other components in the system 100. The DSP may be configured to take signals, such as voice, audio, video, temperature, pressure, position, etc. that have been digitized and then process them as needed. Storage 118 may be configured to store data, software, media, files, and the like, and may include map/navigation data, sensor data and/or other vehicle-related data, discussed in greater detail below. Autonomous driving circuitry 120 may include circuitry and components configured to navigate the vehicle 100 autonomously and/or semi-autonomously based on environment sensing (e.g., via 108). The level of autonomous driving capabilities may be configured according to any of the 6 levels of driving automation defined by the Society of Automotive Engineers (SAE), ranging from Level 0 (fully manual) to Level 5 (fully autonomous).

Display/Haptic circuit 122 may consist of multiple physical displays (e.g., virtual cluster instruments, infotainment or climate control displays) and may further include haptic feedback circuitry configured in a steering wheel, seat, or other suitable location in the vehicle. Display circuit 122 may be configured to provide visual (as well as audio) indicial from any circuitry in FIG. 1, and may be a configured as a human-machine interface (HMI), LCD, LED, OLED, or any other suitable display. The display circuit 122 may also be configured with audio speakers for providing audio output. The display circuit 122 may also include heads-up display (HUD) circuitry for HUD communication. Input/output circuitry 124 may be configured to provide data input and outputs to/from other peripheral devices, such as cell phones, key fobs, device controllers, and the like. As discussed above, circuitries 116-124 may be communicatively coupled to data bus 114 for transmitting/receiving data and information from other circuitries.

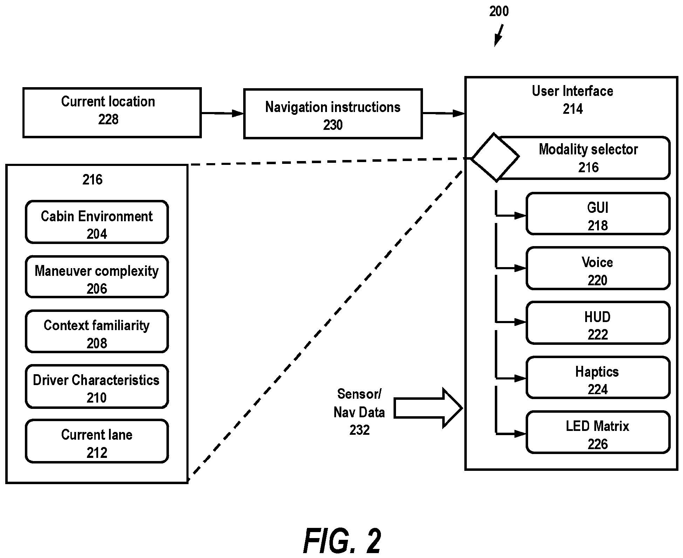

FIG. 2 shows a block diagram illustrating a navigation system 200 including a modality selector 216 and user interface 214 according to some aspects of the present disclosure. In this example, a vehicle (e.g., 102) has a navigation system 200 that receives current location data 228, which is processed to generate navigation instructions 230 as shown in the figure, which is transmitted to a user interface 214 for communication to the driver. The navigation system 200 is configured with a modality selector logic 216, which receives sensor and/or navigation data 232. The sensor data for 232 may include, but is not limited to, data received and processed from physiological sensors, as well as visual and/or audio data of the interior and/or occupant(s) of the vehicle. The physiological data may include, but is not limited to, heart rate (HR) and galvanic skin response (GSR) recordings, instantaneous respiratory rate (IRR), average number of contraction/Minutes (CPM), electrocardiography (ECG) recordings, electromyography (EMG) recordings, and/or electroencephalogram (EEG) recordings. The physiological data may be trained and classified utilizing mechanisms such as an artificial neural network (ANN) model, or any other suitable mechanism. The physiological sensor(s) may be configured to be a part of the vehicle, or may be separately configured on or near a person of the driver (e.g., smart watch, smart phone, or other mobile device/sensor).

Sensor data for 232 may also include image data, such as facial image data, which may be subjected to emotional intelligence algorithms for determining an emotive state of a driver. The emotive state may be determined by periodic capture of a driver's face and comparison to a database for detecting an emotive state. One exemplary database platform for such emotion detection includes AffectNet, which is based on detecting categorical and dimensional models of facial images. In another example, an ASCERTAIN database platform may be used, which is a multimodal database for detecting personality traits and emotional states via facial images and physiological responses. One skilled in the art will recognize that other suitable database platforms (e.g., Dreamer, Extended Cohn-Kanade Dataset (CK+), EMOTIC, etc.) may be utilized, depending on the specific application needed.

In some examples, sensor data for 232 may also include processed audio data for determining an emotive state of a driver. In this example, recorded audio within the vehicle cabin may be subjected to speech emotion recognition (SER) algorithms for detecting an emotive state. In some examples, low-level acoustic speech parameters, or groups of parameters, may be analyzed to determine correlation with the speaker's emotions. The analysis may use standard classifiers such as the Support Vector Machine (SVM), Gaussian Mixture Model (GMM), and shallow Neural Networks (NNs) for such tasks. Alternately or in addition, more complex parameters, such as the Mel-frequency cepstral coefficients (MFCCs), spectral roll-off, Teager Energy Operator (TEO) features, spectrograms, and/or glottal waveform features may be used. The audio data may further be subjected to supervised deep-learning neural network models, or other suitable models, to identify more precise emotive states. One skilled in the art will recognize that other suitable SER configurations may be utilized, depending on the specific application needed. Of course, more simplified techniques may be used to determine a vehicle cabin environment from audio. For example, a microphone may detect a decibel level in the cabin and a processor may assign a cabin environmental characteristic (e.g., loud, semi-noisy, silent) based on the measurement. Also, more specific characteristics (e.g., talking, music playing, passenger sleeping, etc.) may be determined from the audio based on a time-frequency analysis of the captured cabin audio.

One skilled in the art will appreciate that other technologies and techniques for determining characteristics of a driver state and/or vehicle cabin state as sensor data for 232 are contemplated in the present disclosure. For example, motion sensors and/or eye-tracking sensors may also be used alternately or in addition to the sensors discussed herein. Also, one skilled in the art will appreciate that various combinations of sensor data may be utilized, depending on the needs of the application. In some examples, the sensor data may be combined with the navigation data (e.g., route, road characteristic data, traffic data, driving lane data, etc.) when being transmitted to modality selector logic 216. In some examples, the navigation data for 232 may also include external data, such as vehicle-to-everything (V2X) or vehicle-to-infrastructure (V2I) data from a road-side unit (RSU) as well.

In this example, the modality selector logic 216 is illustrated in an expanded block diagram (dotted lines), where it is shown that the modality selector logic 216 is configured with a cabin environment logic 204, which may be configured to determine, using any of the sensor data from 232, one or more cabin environment characteristics. Such characteristics include, but are not limited to, cabin image/audio levels (e.g., light, dark, loud, quiet), and cabin image/audio characteristics (e.g., light direction, presence of illuminated screens, music playing, conversation, singing, sleeping, etc.). These and other characteristics associated with the modality selector logic 216 may be configured automatically, or may be modified via a user, for example, using one or more user preference inputs. Maneuver complexity logic 206 processes navigation data, as well as vehicle data, to determine a complexity level or value along one or more navigational path (e.g., 308) segments. The complexity level may be determined by geometric calculations of navigational path segments, and may be stored as independent values in the navigation system for each navigational path segment. Thus, a straight navigational path may be determined to have a low or minimal complexity level, while navigational paths having many sharp turns along a short path may be determined to have a high complexity value. In some examples, the complexity level may be stored as a dependent value, which relies, for example, on the speed of the vehicle while driving along the navigational path segment. In some examples, a vehicle traveling at a low speed along a moderately complex geometric navigational path may have a lower-to-moderate complexity level. In contrast, the same geometric navigational path, traversed at a much higher speed by the vehicle, may be determined to have a high complexity level. Similar dependencies may be configured for the complexity level using other vehicle driving characteristics (e.g., driving in the day, driving at night).

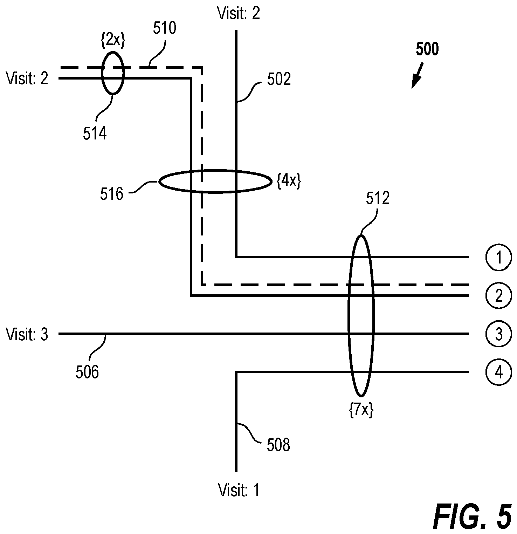

Context familiarity logic 208 may be configured to process navigational data and navigational data histories to determine a driver context familiarity for at least one of a plurality of navigational paths. The processing of navigational data and navigational data histories for context familiarity is described in more detail below, in the example of FIG. 5. Generally, the context familiarly processing determines which navigational paths a driver is most familiar with, in which case the navigational system is configured to modify one or more modalities in which navigational information is communicated. Driver characteristics logic 210 may be configured to process sensor data described herein to determine driver characteristics, such as physiological and/or emotive characteristics. Current lane logic 212 processes navigation data to determine a specific driving lane the vehicle occupies during driving.

In some examples, each recorded sensor data of the driver characteristics (e.g., heart rate, ECG, audio/image emotive state) for driver characteristics module 210 may be processed individually. In some examples, the recorded sensor data may be combined to calculate a value representing overall driver characteristics. Such a configuration may be advantageous in that multiple customized driver characteristics or states may be defined for use in the system 200. Additionally, combinations of driver sensor data values or ranges may simplify processing by combining correlated sensor values over one or more ranges to define a driver characteristic. For example, sensor recordings of a driver heart rate that meets or exceed a configured threshold may be combined with detected facial expression data to define a specific emotive state of the driver. In another example, the detection of one driver characteristic (e.g., heart rate at or above a threshold) may be used to activate data collection from one or more other sensors (e.g., camera for facial expression, GSR detection) to determine an overall driver emotive characteristic. Such a configuration may be advantageous to minimize the need for simultaneous data capture from multiple sensors, and instead provide the ability to sequentially "ladder" sensor data collection to reduce processing and data overhead requirements, as well as energy consumption. Of course, sensor data used for processing in cabin environment logic 204 may also be combined in a similar manner.

Modality selector logic 216 is then configured to dynamically control modalities in the user interface logic 214. The modality selector logic 216 is configured to process individual and/or combined data from any of logics 204-212, to determine a modality in which navigation information is communicated. As used herein, "modality" may be generally defined as a manner, mode, and/or medium in which information is communicated. It should also be understood by one skilled in the art that the processing of data from logics 204-212 to determine one or more modalities may be performed exclusively in the modality selector logic 216, exclusively in another component of the vehicle system (e.g., 116), or performed as a combination of processing steps between modality selector logic 216 and the other vehicle component.

The modality selector logic 216 may be configured to determine one or more modalities based on one or more values, or combinations of values, determined from the sensor data and/or navigation data from any of logics 204-212. The format of the modality value may be configured according to the needs of the system operator. In some examples, the modality value may be an alphanumeric number generated from one or more of the logics 204-212, which represents individual values or combinations of values recorded from logics 204-212. In some examples, the modality value may be configured as a data string, representing individual values or combinations of values recorded from logics 204-212. Once a modality value is determined, the modality selector logic 216 may process the value (e.g., via a look-up table, a relational database, etc.), in which the processed modality value specifies one or more of a plurality of modalities to be used when communicating navigational information. These modalities may include, but are not limited to, a GUI 218, voice communication 220, HUD 222, haptic feedback 224 and/or a LED matrix 226. Based on the modality value, any one, or combinations thereof, of devices 218-226 may be selectively activated and/or deactivated for communicating navigational information. In some examples, the modalities determined by the modality value of modality selector logic 216 may be static (e.g., the modalities remain fixed through the duration of the navigational path). In some examples, the modality selector logic 216 continues to receive further sensor data and/or navigational data from logics 204-212 during traversal of a navigational path, and calculates updated modality values (i.e., dynamic modality) at configured times, in which the modality activation/deactivation of devices 218-226 are also updated if the sensor data and/or navigational data from logics 204-212 changes.

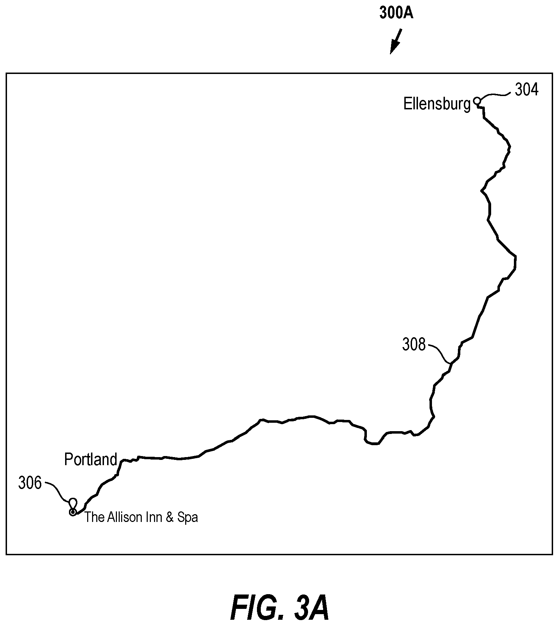

FIG. 3A shows a simulated navigation map 300A including a navigational path 308 between a start point 304 and end point 306 according to some aspects of the present disclosure. This example illustrates the entry of a navigational path into a vehicle navigation system, in which the vehicle navigation system calculates an initial navigational path, as is conventionally known in the art.

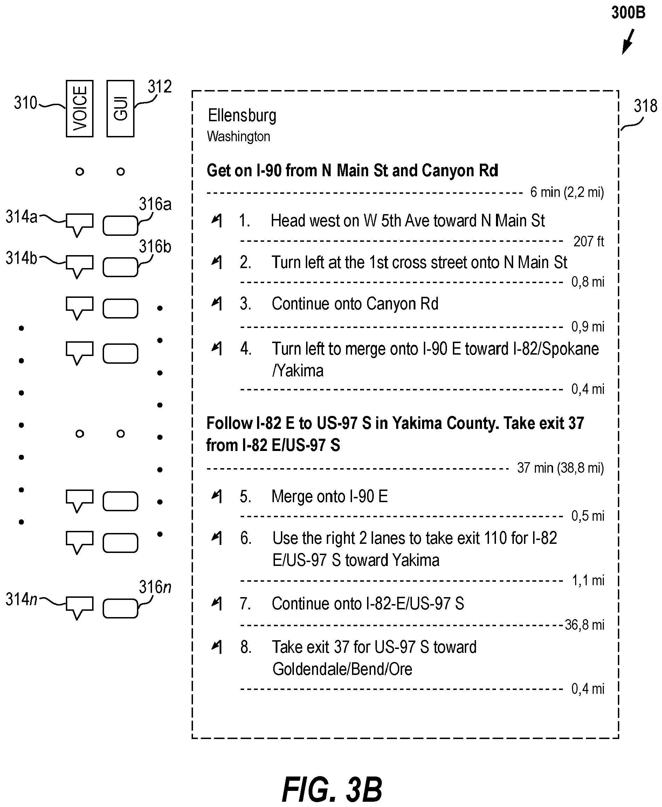

FIG. 3B shows a simulated turn-by turn instruction setting 300B for a navigational system having a static modality according to some aspects of the present disclosure. In this example, the instruction setting 300B is associated with an entered navigational path, such as the one disclosed above in connection with FIG. 3A. In this example, turn-by-turn instructions 318 are provided, giving a textual display of the navigational steps (e.g., 1-8) to traverse at least a portion of a navigational path (e.g., 318). The modalities in this example are selected such that each navigational step is associated with a modality, which in this case includes voice 310 and GUI display 312. As can be seen in the figure, a plurality of activations 314a-314n are assigned for voice modality 310, as well as a plurality of GUI activations 316a-316n, meaning that, for each step 1-8, a voice command and GUI display is activated that displays and announces the navigational step as it is reached.

Thus, for step 1 of this example, a GUI display 316a and voice command 314a would be activated to display the navigational step on the GUI display for the user, and also audibly indicate the first navigational step to the user ("Head west on W 5$^{th}$ Ave towards N Main St"). Similarly, for step 2, a GUI display 316b and voice command 314b would be activated to display the navigational step on the GUI display for the user, and also audibly indicate the second navigational step to the user ("Turn left at the 1$^{st}$ cross street onto N Main St"). This process continues until the last navigational step, in which the GUI display 316n and voice command 314n are activated.

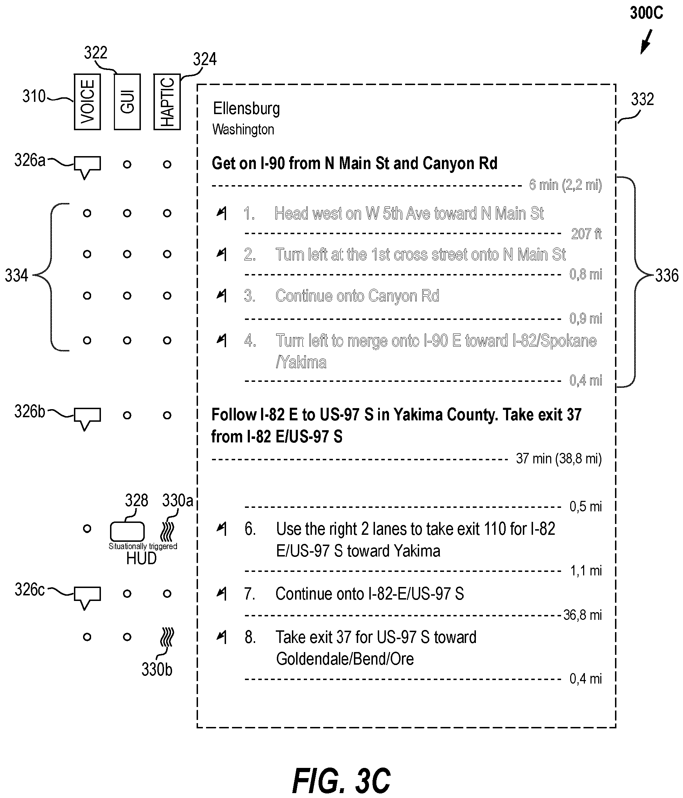

FIG. 3C shows a simulated turn-by turn instruction setting 300C under a dynamic modality configuration according to some aspects of the present disclosure. Similar to the example in FIG. 3B, turn-by-turn instructions 332 are provided, giving a textual display of the navigational steps (e.g., 1-8) to traverse at least a portion of a navigational path (e.g., 318). The modalities in this example are selected such that each navigational step is associated with a modality, which in this case includes voice 320, a GUI display 322 and a haptic response 324. Unlike the example of FIG. 3B, the modalities are configured in the example according to the data processed and generated from modality selector 216, discussed above. Here, the modality selector 216 dynamically changes the modalities for 320-324, such that the activations/deactivations of specific modalities are tied to data recorded and processed from modality selector data 204-212.

Here, based on the processed modality selector data, the voice modality 320 is activated in 326a, 326b, and 326c for their respective navigational path segments, and is deactivated in all other navigational path segments. In this example, the GUI modality 322 is only activated in 328 for the respective navigational path segment, and is otherwise deactivated for all other navigational path segments. Also, haptic feedback modality 324 is activated for 330a and 330b for the associated navigational path segments, and deactivated for all other navigational path segments. As can be seen from the turn-by-turn instructions 1-4 (336), the modality selector 216 deactivates any instructions during these navigational path segments, as well as any associated modalities 334. In a following navigational path segment ("Follow 1-82 to US-97 S in Yakima County. Take exit 37 from 1-82 E/US-97S") the modality selector 216 activates a voice command 326b corresponding with the navigational path segment.

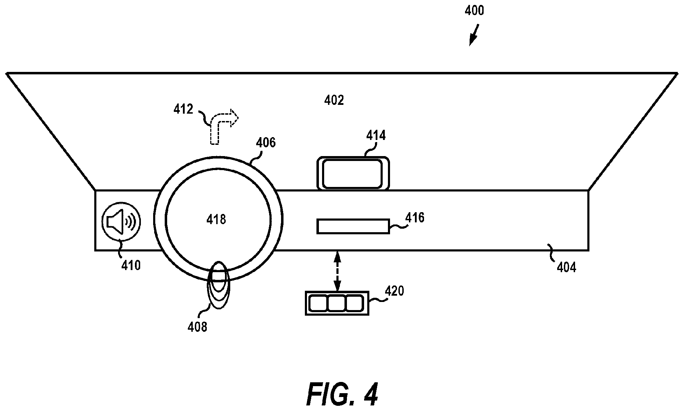

For the following navigational path segment ("Use the right 2 lanes to take exit 110 for I-82 E/LTS-97 S towards Yakima") the modality selector 216 deactivates the voice modality 320 and activates both the GUI 322 and haptic 324 modalities by providing a GUI representation 328 of the navigation path segment instruction, as well as haptic feedback 330a, which may be, for example, a vibrating activation of a haptic actuator in a steering wheel (see 408 of FIG. 4), or other suitable haptic feedback mechanism (e.g., seat vibration). Similarly, for the following navigational path segment, the modality selector 216 deactivates the GUI modality 322 and haptic feedback 324 and activates voice modality 320 to generate a voice command 326c for the associated navigational path segment instruction. The modality selector 216 then deactivates the voice modality 320 and activates the haptic modality 324 to provide haptic feedback 330b for the associated navigational path segment instruction ("Take exit 37 for US-97 S toward Goldendale/Bend/Ore").

Those skilled in the art will appreciate that the present disclosure provides an innovative and elegant configuration for dynamically controlling modalities for vehicle-based navigation. Using sensor data, navigation instructions may be configured to provide such information, based on a driver environment. This, in turn, creates efficiencies in the navigation system, in that navigation instructions are tailored to provide the information using a modality that suits the driver environment, and removes modalities that may not be suited for, or appropriate for, one or more driver environments. In addition to sensor data, navigation data may be used alternately or in addition to provide even further efficiencies. As will be explained in further detail below, driver context familiarity may also be used to determine driver familiarity with navigational paths to filter unnecessary navigational instructions from the navigational system.

FIG. 4 shows a simplified diagram of a vehicle interior 400 including a navigation system and various associated components according to some aspects of the present disclosure. In this example, the vehicle interior 400 includes a windshield 402 and dashboard 404 that includes a display unit 414 that may be part of a navigation system (e.g., 106) configured to generate navigational data. The steering wheel 406 may be configured with a separate display 418 integrated into a face of the steering wheel, as well as a haptic feedback apparatus 408. The vehicle interior 400 may also be configured with a one or more speakers 410, a HUD 412, and/or an LED matrix 416. One or more sensors 420 (e.g., physiological sensors, visual and/or audio sensors, etc.) may be configured to communicate with the vehicle processor to collect sensor data. One having ordinary skill in the art will recognize that the example of FIG. 4 is merely one configuration, and that multitudes of other configurations are contemplated in the present disclosure.

The example of FIG. 4 may be configured such that the different modalities discussed herein may be dynamically activated and/or deactivated to communicate navigation instructions with a driver (or other passengers) occupying the vehicle interior 400. For example, a microphone (e.g., part of sensor system 420) may detect a passenger breathing cadence or snoring within the vehicle interior 400. Using the audio sensor data, the modality selector logic 216 may dynamically change the modality in the user interface 214 such that audio instructions (e.g., via speaker 410) are deactivated. In addition, the modality selector logic 216 may activate other modalities (e.g., LED matrix 416, display(s) 414/418, HUD 412) alone or in combination to compensate for the deactivation of audio instructions. In some examples, the dynamic modality selection may also be configured to interact with autonomous driving circuitry (120) to activate/modify/deactivate levels of autonomy in the vehicle driving capabilities.

FIG. 5 shows an illustration of a navigational map 500 having multiple available navigational paths (1-4), along with a selected navigational path 510, in which a familiarity score is calculated for configuring navigation instructions according to some aspects of the present disclosure. In this example, the four navigational paths include a first navigational path, 502, a second navigational path, 504, a third navigational path 506 and a fourth navigational path 508. A navigational path 510 is then selected by a driver (shown as a dashed line in the figure), which coincides with navigational path 504. Prior to navigating the vehicle, the navigation system (e.g., 106) may calculate a driver route familiarity for each of the navigational path segments.

In this example, the navigation system determines that for the navigation path segment 512, the driver has traversed this portion seven times (" {7x} "), and for navigation path segment 516, the driver has traversed this portion four times (" {4x} "). For navigation path segment 514, the navigation system determines that the driver has traversed this navigation path segment two times ("{2x}"). In some examples, the driver route familiarity may be configured such that a complete route selection must be visited at least 2 times in order to be considered for familiarity processing. Such configurations may be advantageous in limiting familiarity processing only to previously-traversed paths, instead of processing all possible rout combinations.

Given the selected navigational path 510, it can be seen that the first navigational path 502 has two previous visits, the second navigational path 504 has two previous visits, the third navigational path 506 has three previous visits, and the fourth navigational path 508 has one visit. In this example, the fourth navigational path 508 is filtered from route familiarity processing, since the path does not meet the two-visit limitation. Accordingly, a route familiarity score may be determined as a function of total previous route visits, previous route segment visits, and total previous route visits for a selected path in the navigation system, according to the following formula:

$$Route\ familiarity = \frac{sum\ of\ (tot.\ route\ visits\ \times\ segment\ visits)}{no.\ of\ tot.\ route\ segments\ for\ selected\ path}$$

[EQ1]

Thus, for the example in FIG. 5, for navigation path segment 512, the route visit along navigation path 506 is three, the route visits for navigation path segment 516 is four (2 visits for path 504, and 2 visits for path 502), and for navigational path segment 514, the route visit along the end of the navigational path is two. Accordingly, the route familiarity score may be expressed as

$$Route\ familiarity = \frac{(3 \times 7 + 4 \times 4 + 2 \times 2)}{3 + 4 + 2}$$

$$Route\ familiarity = \frac{41}{9} = 4.55 = 45.5\%$$

Using the route familiarity score, alone or in combination with other sensor data (e.g., 204-206, 210-212), the modality selector logic 216 may be configured to dynamically activate and/or deactivate modalities in the navigation instructions. Thus, for navigational paths or path segments having high user familiarity, the navigation system may deactivate one, some, or all notifications for paths or segments having a high familiarity score. One skilled in the art will recognize that a threshold route familiarity score may be dynamically set, depending on the specific application. For example, the navigation system may be set such that navigational information is deactivated only for navigational paths or path segments having a familiarity score of 100%. In some examples, the deactivation threshold may be set lower (e.g., 80%). In some examples, various modalities may activated/deactivated based on predetermined familiarity thresholds in a laddered configuration (e.g., activating/deactivating modalities based on <50%, 75%, and 100% familiarity).

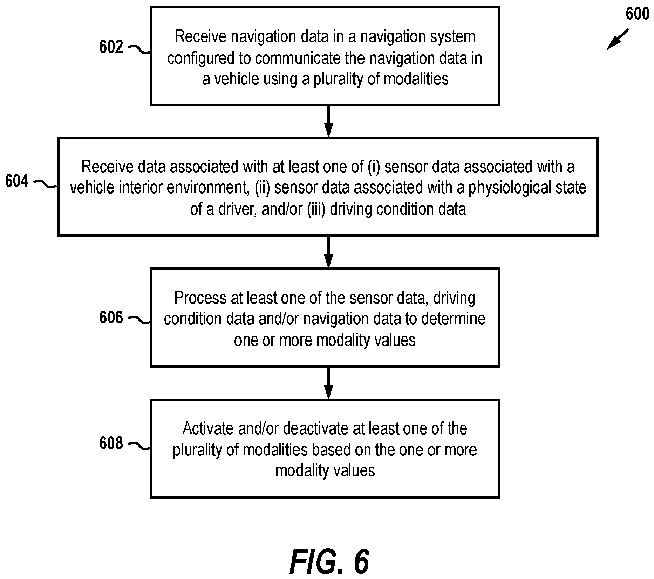

FIG. 6 shows an illustrative process 600 for dynamically controlling modalities for vehicle navigation, according to some aspects of the present disclosure. In block 602, navigation data may be received in a navigation system (e.g., 106) configured to communicate the navigation data in a vehicle using a plurality of modalities (e.g., 218-226). In block 604, a processor (e.g., 106, 116) may receive data associated with at least one of sensor data associated with a vehicle interior environment (e.g., cabin environment 204), sensor data associated with a physiological state of a driver (e.g., 210), and/or driving condition data (e.g., 206, 208, 212). In block 606 the processor may process at least one of the sensor data, driving condition data and/or navigation data to determine one or more modality values (e.g., 216, 216). In block 608, at least one of the plurality of modalities may be activated and/or deactivated (e.g., via 216 and 218-226) based on the one or more modality values.

In some examples, the vehicle interior environment may include audio sensor data, and/or image sensor data associated with the vehicle interior. Data associated with the physiological state of the driver may include at least one of physiological sensor data, image sensor data, and/or audio sensor data. In some examples, the driving condition data may include vehicle-to-everything (V2X) or vehicle-to-infrastructure (V2I) data. In some examples, the driving condition data may include at least one of maneuver complexity level data (e.g., 206), current lane data (e.g., 212), and context familiarity data (e.g., 208). The context familiarity data may include a route familiarity value (FIG. 5) representing a driver's familiarity with one or more navigational route segments. The route familiarity value may be calculated as a function of total previous route visits, previous route segment visits, and total previous route visits for a selected path in the navigation system (e.g., [EQ1]).

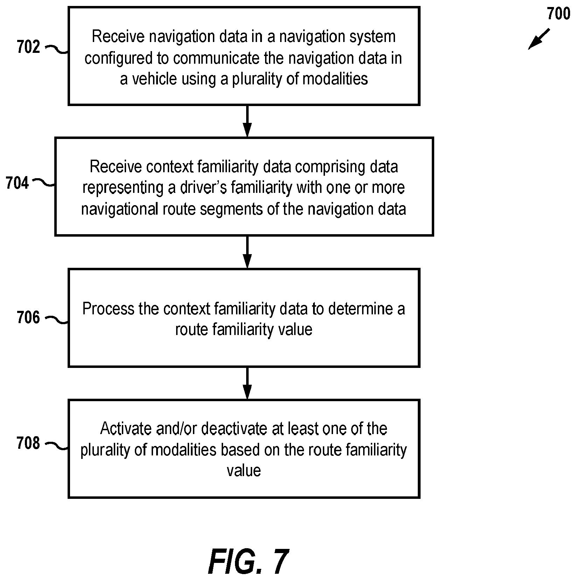

FIG. 7 shows another illustrative process 700 for dynamically controlling modalities for vehicle navigation using route familiarity, according to some aspects of the present disclosure. In block 702, navigation data in a navigation system may be received, wherein the navigation data is configured to communicate the navigation data in a vehicle using a plurality of modalities (e.g., 218-226). In block 704, context familiarity data (e.g., 208) may be received comprising data representing a driver's familiarity with one or more navigational route segments of the navigation data (FIG. 5). In block 706, the context familiarity data may be processed to determine a route familiarity value ([EQ1]). In block 708, at least one of the plurality of modalities may be activated/deactivated (e.g., via 216 and 218-226) based on the route familiarity value.

In some examples, the route familiarity value may be calculated as a function of total previous route visits, previous route segment visits, and total previous route visits for a selected path in the navigation system (e.g., [EQ1]). The process 700 may further include receiving sensor data associated with at least one of a vehicle interior environment (e.g., 204), and a physiological state of a driver (e.g., 210), and activating and/or deactivating (e.g., via 216) the at least one of the plurality of modalities (e.g., 218-226) based on the sensor data. In some examples, the vehicle interior environment may include audio sensor data, and/or image sensor data associated with the vehicle interior. In some examples, the physiological state of the driver may include at least one of physiological sensor data, image sensor data, and/or audio sensor data. The process 700 may further include receiving driving condition data comprising at least one of maneuver complexity level data (e.g., 206) and current lane data (e.g., 212), and activating and/or deactivating (e.g., via 216) at least one of the plurality of modalities (e.g., 218-226) based on the driving condition data.

Under the configurations and examples provided herein, one skilled ion the art will recognize that the present disclosure provides technologies and techniques for making turn-by-turn navigation more situationally intelligent and relevant by orchestrating the communication modalities depending on a current driving condition or environment in relation to the driver.

Conventional turn-by-turn instructions typically provide instructions via all connected interior modalities at all times (e.g., GUI/visuals, HUD, voice commands) with little to no regard to the driver's familiarity with the driving area and perceived complexity of the driving task, which in turn may be a function of driver's current cognitive load and stress level. The present disclosure provides a more contextual aware system that can orchestrate the most relevant modalities to minimize a potential overflow of information. As innovations and new technologies for car interiors advance, ways of communication information to the user expand. To ensure a better user experience that isn't overly demanding on a driver's cognition, the suitable communication modalities should be selected, tailored for particular driving conditions.

The present disclosure thus reduces the cognitive load of navigation by reducing the amount of situationally unnecessary information given to the driver and/or providing more supporting instructions for complex maneuvers. Alternately or in addition, the present disclosure improves a user experience and driving enjoyment by creating a more calm and ambient driving experience. Based on contextual data points (e.g., real-time cabin mood measurements, complexity of a current maneuver, and/or the driver's familiarity with the context), the system may determine the appropriate conveyance modality combination.

As described above, the disclosed technologies and techniques may use a combination of data points to decide the right communication modality. These data points include, but are not limited to, contextual data about surroundings (measured data) that include traffic data, driving lane data and road data (e.g., V2X RSU data). The data points may further include cabin environment data, driver's trip data (inferred data), and driver route familiarity. The driver route familiarity, as discussed above. In some examples, the route familiarity value may also be weighted, depending on whether the navigation route segment is a highway, expressway, inner-street, etc. Navigation Data Standard (NDS) categories and waypoints may also be utilized to denote what type of street a route belongs to.

Further data points may include driver physiology, which may include driver stress levels, where a driver's current stress level may be taken into account using physiological sensor measurements, where excess stress levels may lead to higher cognitive load for a given navigational task. As discussed above, stress levels may be measured, in some examples, using a combination of electrodermal activity (e.g., micro-sweat in user's palm) using sensors from a steering wheel of smart watch. Additionally, high-rate variability may be measured during driving using sensors configured in a seat and/or seat belt. Cabin interaction level can be detected as a combination of a plurality of inferred signals (e.g., sleep detection, conversation detection, loudness/movement detection). Conversation detection can be implemented using in-cabin microphone array(s), and cabin loudness could be configured as a sub-category of conversation detection, where cabin loudness may be determined using a combination of microphone arrays, as well as cabin monitoring movement sensors. In some examples, passenger (front and back seat) sleep detection may be implemented using face and eye-tracking camera(s), and/or radar movement sensor, which can detect, for example, approximately 2 mm movements/vibrations in a vehicle cabin.

Under the present disclosure, when drivers are driving in their own neighborhood, they typically do not need detailed instructions for every maneuver, but can simply be told to get on a highway in a certain direction. However, for a driver unfamiliar with the area, the navigation system wouldn't leave out these details. Alternately or in addition, if a driver is instructed to take an exit that requires the vehicle to be in a certain lane, that information may be displayed, for example, in the HUD. Conversely, if this information isn't essential to successfully maneuver the vehicle correctly, no information will be displayed in the HUD. If a driver is not in the correct lane to make an upcoming exit, the system may combine haptic vibrations in the steering wheel (to get the driver's attention) in addition to visuals displayed in the HUD, preferably with detailed instruction on the correct lane position.

Alerts could also be used in instances where the driver does not have an active navigation running, but where the system has a configured prediction that the driver is about to miss their exit (e.g., during morning commutes, where the predictive navigation assumes the driver is going to their work destination). Also, the navigation system may mute modalities if someone is asleep in the vehicle. For example, if a child falls asleep during a trip, the system may be configured to automatically change the modalities to be as least intrusive as possible. Specifically, the system may change modalities to avoid the usage of voice and auditory cues. The navigation system may also be configured to indicate automatic changes via one or the modalities (e.g., indicating on display that audio has been turned off). Of course, the system may be configured to allow drivers to override the changes for individual preferences.

Aspects of the present disclosure are described in terms of turn-by-turn navigation, but those skilled in the art should appreciate that the present disclosure may also be applied to other use cases for communicating driving instructions to the driver, such as hand-overs from autonomous driving, parking sensors, etc. The data associated with cognitive load measurement may also be used to control a "modality volume" on other non-driving notifications, such as non-driving but time-critical notifications (e.g., phone call, text message), and/or non-driving and non-time-critical notifications (e.g., song change, in-car gaming notifications).

As described above, some or all illustrated features may be omitted in a particular implementation within the scope of the present disclosure, and some illustrated features may not be required for implementation of all examples. In some examples, the methods and processes described in FIGS. 6-7 may be performed by a vehicle (e.g., 102), as described above and/or by a component, processor/processing system or circuitry (e.g., 104-124) or by any suitable means for carrying out the described functions.

**Claims**

1. A system (100) for controlling modalities (218-226) for vehicle navigation, comprising:

   a navigation system (106, 200) configured to transmit navigation data (230, 232) and communicate the navigation data (230, 232) in a vehicle (102) using a plurality of modalities (218-226);
   one or more sensors (108) configured to provide (604) data (232) associated with at least one of (i) sensor data associated with a vehicle interior environment, (ii) sensor data associated with a physiological state of a driver, and/or (iii) driving condition data; and
   a processor (116, 216), operatively coupled to the one or more sensors (108) and the navigation system (106, 200), wherein the processor (116, 216) is configured to process (606) at least one of the sensor data, the driving condition data, and/or the navigation data to determine one or more modality values, wherein the processor (116, 216) is configured to activate and/or deactivate (608) at least one of the plurality of modalities (218-226) based on the one or more modality values.

2. The system (100) of claim 1, wherein the sensor data associated with the vehicle interior environment comprises audio sensor data and/or image sensor data associated with the vehicle interior.

3. The system (100) of claim 1 or 2, wherein the sensor data associated with the physiological state of the driver comprises at least one of physiological sensor data, image sensor data, and/or audio sensor data.

4. The system (100) of one of the preceding claims, wherein the driving condition data comprises vehicle-to-everything, V2X, or vehicle-to-infrastructure, V2I, data.

5. The system (100) of one of the preceding claims, wherein the driving condition data comprises at least one of maneuver complexity level data, current lane data, and/or context familiarity data.

6. The system (100) of claim 5, wherein the context familiarity data comprises a route familiarity value representing a driver's familiarity with one or more navigational route segments.

7. The system (100) of claim 6, wherein the route familiarity value is calculated as a function of total previous route visits, previous route segment visits, and total previous route visits for a selected path (510) in the navigation system (106, 200).

8. A method (600) for controlling modalities (218-226) for vehicle navigation, comprising:

   receiving (602) navigation data (230, 232) in a navigation system (106, 200) configured to communicate the navigation data (230, 232) in a vehicle (102) using a plurality of modalities (218-226);
   receiving (604) data associated with at least one of (i) sensor data associated with a vehicle interior environment, (ii) sensor data associated with a physiological state of a driver, and/or (iii) driving condition data;
   processing (606), via a processor (116, 216), at least one of the sensor data, the driving condition data, and/or navigation data to determine one or more modality values; and
   activating and/or deactivating (608) at least one of the plurality of modalities (218-226) based on the one or more modality values.

9. The method (600) of claim 8, wherein the sensor data associated with the vehicle interior environment comprises audio sensor data and/or image sensor data associated with the vehicle interior.

10. The method (600) of claim 8 or 9, wherein the sensor data associated with the physiological state of the driver comprises at least one of physiological sensor data, image sensor data, and/or audio sensor data.

11. The method (600) of one of claims 8 to 10, wherein the driving condition data comprises vehicle-to-everything, V2X, or vehicle-to-infrastructure, V2I, data.

12. The method (600) of one of claims 8 to 11, wherein the driving condition data comprises at least one of maneuver complexity level data, current lane data, and/or context familiarity data.

13. The method (600) of claim 12, wherein the context familiarity data comprises a route familiarity value representing a driver's familiarity with one or more navigational route segments.

14. A method (700) for controlling modalities (218-226) for vehicle navigation, comprising:

receiving (702) navigation data (230, 232) in a navigation system (106, 200) configured to communicate the navigation data (230, 232) in a vehicle (102) using a plurality of modalities (218-226);
receiving (704) context familiarity data comprising data representing a driver's familiarity with one or more navigational route segments of the navigation data (230, 232);
processing (706), via a processor (116, 216), the context familiarity data to determine a route familiarity value; and
activating and/or deactivating (708) at least one of the plurality of modalities (218-226) based on the route familiarity value.

15. The method (600, 700) of claim 13 or 14, wherein the route familiarity value is calculated as a function of total previous route visits, previous route segment visits, and total previous route visits for a selected path (510) in the navigation system (106, 200).

FIG. 1

FIG. 2

**300A**

FIG. 3A

**300B**

**310** VOICE GUI **312**

**314a** **316a**
**314b** **316b**

**314n** **316n**

**318**

Ellensburg
Washington

**Get on I-90 from N Main St and Canyon Rd**
-------------------------------------------------- 6 min (2,2 mi)

1. Head west on W 5th Ave toward N Main St
-------------------------------------------------- 207 ft

2. Turn left at the 1st cross street onto N Main St
-------------------------------------------------- 0,8 mi

3. Continue onto Canyon Rd
-------------------------------------------------- 0,9 mi

4. Turn left to merge onto I-90 E toward I-82/Spokane /Yakima
-------------------------------------------------- 0,4 mi

**Follow I-82 E to US-97 S in Yakima County. Take exit 37 from I-82 E/US-97 S**
-------------------------------------------------- 37 min (38,8 mi)

5. Merge onto I-90 E
-------------------------------------------------- 0,5 mi

6. Use the right 2 lanes to take exit 110 for I-82 E/US-97 S toward Yakima
-------------------------------------------------- 1,1 mi

7. Continue onto I-82-E/US-97 S
-------------------------------------------------- 36,8 mi

8. Take exit 37 for US-97 S toward Goldendale/Bend/Ore
-------------------------------------------------- 0,4 mi

## FIG. 3B

**300C**

**322** **324**

**310**

VOICE | GUI | HAPTIC

**326a**

**334**

**326b**

**328 330a**

Situationally triggered
**HUD**

**326c**

**330b**

**332**

**336**

Ellensburg
Washington

**Get on I-90 from N Main St and Canyon Rd**

------ 6 min (2,2 mi)

1. Head west on W 5th Ave toward N Main St

------ 207 ft

2. Turn left at the 1st cross street onto N Main St

------ 0,8 mi

3. Continue onto Canyon Rd

------ 0,9 mi

4. Turn left to merge onto I-90 E toward I-82/Spokane
/Yakima

------ 0,4 mi

**Follow I-82 E to US-97 S in Yakima County. Take exit 37
from I-82 E/US-97 S**

------ 37 min (38,8 mi)

------ 0,5 mi

6. Use the right 2 lanes to take exit 110 for I-82
E/US-97 S toward Yakima

------ 1,1 mi

7. Continue onto I-82-E/US-97 S

------ 36,8 mi

8. Take exit 37 for US-97 S toward
Goldendale/Bend/Ore

------ 0,4 mi

## FIG. 3C

**FIG. 4**

FIG. 5

600

602 — Receive navigation data in a navigation system configured to communicate the navigation data in a vehicle using a plurality of modalities

604 — Receive data associated with at least one of (i) sensor data associated with a vehicle interior environment, (ii) sensor data associated with a physiological state of a driver, and/or (iii) driving condition data

606 — Process at least one of the sensor data, driving condition data and/or navigation data to determine one or more modality values

608 — Activate and/or deactivate at least one of the plurality of modalities based on the one or more modality values

*FIG. 6*

**700**

702 — Receive navigation data in a navigation system configured to communicate the navigation data in a vehicle using a plurality of modalities

704 — Receive context familiarity data comprising data representing a driver's familiarity with one or more navigational route segments of the navigation data

706 — Process the context familiarity data to determine a route familiarity value

708 — Activate and/or deactivate at least one of the plurality of modalities based on the route familiarity value

*FIG. 7*

Europäisches
Patentamt

European
Patent Office

Office européen
des brevets

## EUROPEAN SEARCH REPORT

Application Number

EP 22 21 0398

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2017/355377 A1 (VIJAYA KUMAR VIVEK [US] ET AL) 14 December 2017 (2017-12-14) * paragraphs [0223] - [0238] * ----- | 1-5,8-12 | INV. G01C21/34 B60W40/09 B60W50/00 G01C21/36 |
| X | US 2016/076903 A1 (DIAZ ANA LILIA OTERO [US] ET AL) 17 March 2016 (2016-03-17) * paragraph [0048] * * paragraphs [0021] - [0033] * ----- | 1,5-8, 12-15 | |

TECHNICAL FIELDS
SEARCHED        (IPC)

G01C
B60W

The present search report has been drawn up for all claims

| Place of search | Date of completion of the search | Examiner |
|---|---|---|
| The Hague | 1 May 2023 | Gagin, Thibaut |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons
 
& : member of the same patent family, corresponding
    document

EPO FORM 1503 03.82 (P04C01)

**ANNEX TO THE EUROPEAN SEARCH REPORT
ON EUROPEAN PATENT APPLICATION NO.**

EP 22 21 0398

This annex lists the patent family members relating to the patent documents cited in the above-mentioned European search report.
The members are as contained in the European Patent Office EDP file on
The European Patent Office is in no way liable for these particulars which are merely given for the purpose of information.

01-05-2023

| Patent document cited in search report | Publication date | Patent family member(s) | | Publication date |
|---|---|---|---|---|
| US 2017355377 A1 | 14-12-2017 | NONE | | |
| US 2016076903 A1 | 17-03-2016 | CN | 106687767 A | 17-05-2017 |
| | | EP | 3191796 A1 | 19-07-2017 |
| | | US | 2016076903 A1 | 17-03-2016 |
| | | WO | 2016040712 A1 | 17-03-2016 |

EPO FORM P0459

For more details about this annex : see Official Journal of the European Patent Office, No. 12/82